# EUROPEAN PATENT SPECIFICATION

(11) **EP 0 551 439 B1**
(45) Date of publication and mention of the grant of the patent: **09.08.1995**
(21) Application number: 91920409.9
(22) Date of filing: 16.09.1991
(51) Int. Cl.: A61F 9/00

(54) **APPARATUS FOR SURGICALLY PROFILING THE CORNEA USING VACUUM**
VORRICHTUNG ZUM CHIRURGISCHEN PROFILIEREN DER HORNHAUT UNTER VAKUUM
APPAREIL DE PROFILAGE CHIRURGICAL DE LA CORNEE AU MOYEN DU VIDE

(30) Priority: 04.10.1990 US 592601
(43) Date of publication of application: 21.07.1993
(73) Proprietor: CORNEAL CONTOURING DEVELOPMENT, L.L.C., Tulsa, Oklahoma 74104 (US)
(72) Inventor: KILMER, Lauren, G., Tulsa, OK 74105 (US); REYNOLDS, Alvin, E., Tulsa, OK 74135 (US)
(74) Representative: Kovacs, Paul
(86) International application number: US9106691
(87) International publication number: WO9210152

(56) References cited:
- EP-A- 0 248 569
- EP-A- 0 303 174
- WO-A-89/00404
- DE-A- 3 707 004
- US-A- 3 797 921
- US-A- 4 526 171

## Description

### BACKGROUND OF THE INVENTION

This invention relates to an apparatus for adjusting the shape of components of the eye and more particularly to making fixed changes in the corneal curvature to correct refractive error.

Deviations from the normal shape of the corneal surface produce errors of refraction in the visual process. The eye in a state of rest, without accommodation, focuses the image of distant objects exactly on the retina. Such an eye enjoys distinct vision for distant objects without effort. Any variation from this standard constitutes ametropia, a condition in which the eye at rest is unable to focus the image of a distant object on the retina. Hyperopia is an error of refraction in which, with the eye at rest, parallel rays from distant objects are brought to focus behind the retina. Divergent rays from near objects are focused still further back. In one aspect of hyperopia, the corneal surface is flattened which decreases the angle of refraction of rays as they pass through the refractive surfaces of the cornea, causing a convergence or focus of the rays at a point behind the retina. The retina is comprised partially of nerve fibers which are an expansion of the optic nerve. Waves of light falling on the retina are converted into nerve impulses and carried by the optic nerve to the brain to produce the sensation of light. To focus parallel rays on the retina, the hyperopic eye must either accommodate, i.e., increase the convexity of its lens, or a convex lens of sufficient strength to focus rays on the retina must be placed before the eye.

Myopia is that refractive condition in which, with accommodation completely relaxed, parallel rays are brought to focus in front of the retina. One condition which commonly causes myopia is when the corneal curvature is steepened, thus the refraction of rays is greater as the rays pass through the refractive surfaces of the cornea, and the over-refracted rays converge or focus in front of the retina in the vitreous of the eye. When the rays reach the retina they become divergent, forming a circle of diffusion and consequently a blurred image. A concave lens is used to correct the focus of the eye for myopia.

The normal treatment of these classic forms of refractive error of the eye is with the use of eyeglasses or contact lenses, both of which have well-known disadvantages to the user. It has been estimated that 60 million pairs of eyeglasses and 3 million pairs of contact lens are sold annually.

Recent research has been directed to operative techniques to change the refractive condition of the eye. Such techniques are generally referred to as "keratorefractive techniques". Two such techniques are more particularly called keratophakia and keratomileusis. Keralomileusis involves the regrinding of a corneal lamella into a meniscus or hyperopic lens to correct myopia or hyperopia. A corneal optical lathe has been especially developed for this procedure and is also used in the keratophakia procedure, when a homograft ground into a convex lens is placed interlamellarly to correct aphakic hypermetropia. The homograft tissue (corneal lamella) is frozen with carbon dioxide. The homograft is cut as a contact lens would be, i.e., to the optical power required to effect the desired optical correction of the cornea. In keratomileusis, the anterior corneal lamella is shaped by the lathe and in keratophakia it is the corneal stroma of a donor eye that is shaped by the lathe. These techniques have a broad application in the correction of high hyperopic and myopic errors. These procedures require radial cutting of the cornea about the periphery of the graft which weakens the cornea so that pressure from fluids below the incisions pushes up under the cuts and flattens the curvature of the cornea. This flattening of the cornea results in refractive errors to the eye not compensated for by the graft. Suturing in these operations also causes radial asymmetry of the cornea which consequently promotes astigmatic error in this regard. Sutures also cause scarring of the corneal tissue, which scar tissue loses its transparency. Surgical correction of astigmatism is accomplished by asymmetrically altering the corneal curvatures. The effect of a peripheral distorting force may be easily visualized by imagining an inflated balloon with a spherical surface being compressed between the palms of the hands. Because the volume of air in the balloon is constant, the surface area remains constant. The previously spherical anterior surface is distorted meridional as a result of compressing the diameter between the hands so that the curvature changes without changing the circumference of the surface. The meridian passing over the balloon between the extended fingers steepens, while the uncompressed meridian at right angles thereto flattens as its diameter lengthens in proportion to the shortening of the compressed diameter. This demonstrates the effect that may result from slight variations in the symmetrical patterns or intentional asymmetrical patterns attempted to be accomplished during surgical procedures and attendance suturing. It is thus seen that present procedures in keratorefractive techniques are best limited to situations where other more standard corrective practices are found ineffective. It is readily seen that the limiting factors in such surgical techniques is the gross complexity involved not only with multiple incisions in corneal tissue for affecting the procedures but also complex suturing patterns, resulting in gross restructuring of the eye. The eye is thus faced with a difficult job of adjusting to this trauma.

Over the past few years developments have been made in the use of lasers as a means to reshape the cornea in an attempt to get rid of refractive errors. In these processes, pulsed lasers remove tissue from the cornea by shaving off or vaporizing portions of the corneal surface to cause it to flatten. The most common type is an Exemer laser. The fundamental effect of such a laser on tissue is a photochemical one, the breaking of molecular bonds with so much energy that the tissue fragments fly from the surface at supersonic speeds, leaving behind a discreet space. The process has been designated as ablative photodecomposition or photoablation.

The use of Exemer lasers require delivery of the beam to the eye in a controlled manner requiring that the homogenous beam be appropriately managed and focused because the optical elements must withstand the high energy photons and because the beam must be shaped to a non-uniform configuration to create the new non-uniform optical surface of the cornea. Such delivery system contains multiple components including lenses to expand or focus the beam, mirrors to direct the beam, modulators to homogenize the beam, masks to shape the beam, and detectors to measure the intensity and configuration of the beam. Current models range from a simple collection of lenses and masks to complex robots with components that control not only the laser parameters but also the optical and mechanical components. Because the process is dealing with submicron (less than .00001 of a meter) accuracy, great demands are placed upon such systems for stability, even though the interaction of the laser and tissue lasts only microseconds.

Using the system requires exquisite technical and biological control to modulate corneal shaping.

Another laser treatment process focuses light, like a magnifying glass, to boil away tissue one cell at a time, instead of carving away the surface. One problem is adequate control to prevent the process from cutting through a layer of corneal tissue known as Bowman's membrane--a section of the eye that does not regenerate.

A preformed grinder cup, with a concave saucer-like surface on the underside which is provided with a fine-grain crystalline and preferably diamond dust covered surface, is disclosed in EP-A-303 174. High-speed grinding technology, as the one used in the apparatus according to said document, requires cooling and a viscoelastic substance as cooling medium is used therein. EP-A-303 174 discloses the preamble of claim 1.

Moreover, when a rotary cup is fed straight down, no cutting action occurs at its rotary center because the radial forces necessary to cause a sweeping and cutting action do not exist at the center of rotation.

A corneal vacuum trephine system for cutting out a piece of the cornea (not scraping it), the trephine being in a hollow-cylinder form, is disclosed in DE-A-3 707 004.

None of these prior art documents discloses or suggests an apparatus allowing the above-mentioned problems to be overcome.

### SUMMARY OF THE INVENTION

It is therefore an object of the present invention to provide an apparatus for carrying out a new and improved keratorefractive technique involving changing the shape of the optical zone of the cornea to correct refractive errors of hyperopia (far-sightedness), myopia (near-sightedness), and astigmatism, whereby a non-spherical surface exists on the eye system and the simplicity of the technique virtually eliminate the chance of error or further complications resulting from gross disturbances of the eye system.

With this and other objects in view, the present invention contemplates an apparatus for carrying out a method that can only be described as scraping, sculpting, or removing portions of the cornea for the purposes of correcting refractive error in human cornea. For the purposes of this invention and that of said co-pending application, S.N. 450,672, the action will be called "scraping".

Another object of the invention is to provide mechanical apparatus capable of easily being used by a surgeon for scraping the cornea in order to correct refractive errors of hyperopia, myopia, and astigmatism which includes means to provide consistency in depth and configuration of the surface.

Another object of this invention is to provide an apparatus for scraping the cornea wherein the cornea is maintained in a more rigid posture during the procedure to eliminate flexure of the cornea and thus provide greater accuracy in determining predicable amounts of corneal material to be removed. This is accomplished by creating a vacuum in the operative space above the corena during the process.

Specifically, the method which can be carried out using the apparatus provided by this invention involves the surgical reprofiling of the corneal portion of an eye of humans, to change the corneal radius and thus correct refractive errors. The steps include creating a placido ring keratograph of a simulated cornea having the correct refractive qualities. Next, an actual keratograph of the cornea is created. The two kerotographs are compared to determine the amount of refractive error, i.e. whether it would be hyperopia, myopia, or astigmatism.

A reprofiling tool is constructed to include a plurality of scraper blades of shape sufficient to change a corneal radius to that of the simulated cornea. The reprofiling tool is then positioned within a holding sleeve that is contiguously positioned upon said eye such that the scraper blades will contact the cornea. A vacuum is created in the chamber above the cornea wherein the scraping tool is positioned. The scraping tool is then rotated or oscillated with the axial movement of the scraping tool being changed and indexed until the corneal radius has been corrected to that of the simulated or ideal cornea.

The apparatus used to achieve the objects of this invention specifically includes a cylindrical positioning ring having a resilient vacuum ring means on its bottom side for temporary attachment to the sclera portion of an eye which surrounds the cornea that is to be reprofiled. A plurality of positioning pins exist on the top side of the positioning ring and a vacuum means is provided for communication with the vacuum ring. A cylindrical holding sleeve includes means at the bottom of the holding sleeve to interconnect with the positioning pins of the cylindrical positioning ring. A flexible and preferably clear tubing member extends from the bottom of the holding sleeve to seal against the cornea. Fine Screw threads of a given pitch, preferably about 40 threads per inch, are formed on the interior or exterior portion of the holding sleeve. Threadably connected thereto is a guide sleeve having screw threads of the same pitch as the threads of the holding sleeve for rotatable attachment with the holding sleeve. A scraping tool is adapted to be rotatably and axially received within the positioning ring, the holding sleeve, and the guide sleeve. A seal means is provided between the scraping tool, the guide sleeve and/or holding sleeve. A collar means existing on the scraping tool allows it to be rotatably supported upon the guide sleeve. A plurality of blades at the bottom of the scraper tool are designed to be of a shape sufficient to scrape away portions of the cornea to achieve the desired corrective curvature.

### BRIEF DESCRIPTION OF THE DRAWINGS

Figure 1 is a schematic illustration of a horizontal section of the eye.

Figure 2 is a schematic illustration of a hyperopic eye showing adjustment of the cornea to shorten the radius of curvature.

Figure 3 is a schematic illustration of a myopic eye system showing adjustment of the cornea to increase its radius and thus flatten the corneal slope.

Figure 4 is a detailed schematic illustration of a horizontal section of the frontal portion of an eye showing the various layers of the cornea.

Figure 5 is an exploded view showing the basic components of the apparatus of this invention.

Figure 6 is an end view of the reprofiling tool taken along the line 6-6 of Figure 5.

Figure 7 is a top view of the tool holder taken along the line 7-7 of Figure 5.

Figure 8 is a top view of the positioning ring taken along the line 8-8 of Figure 5.

Figure 9 is an assembly view, partly cut-away to show the apparatus of the invention.

### DETAILED DESCRIPTION OF THE PREFERRED EMBODIMENT

Before explaining the present invention in detail, it is to be understood that the invention is not limited in its application to the details of the construction and arrangement of parts illustrated in the accompanying drawings. The invention is capable of other embodiments and of being practiced or carried out in a variety of ways. It is to be understood that the phraseology and terminology employed herein is for the purpose of description and not of limitation.

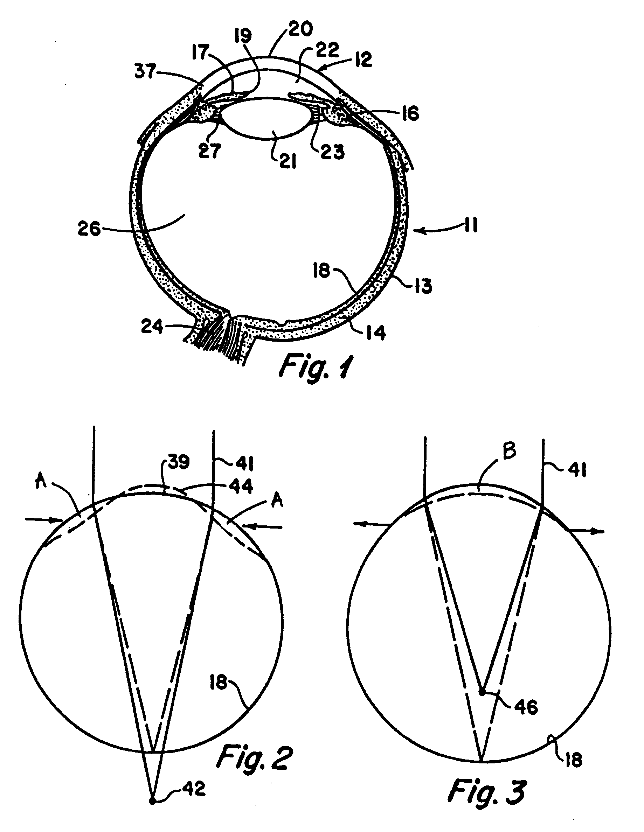

Referring first to Figure 1 of the drawings, a horizontal section of the eye shows the globe of the eye resembling a sphere with an anterior bulged spherical portion 12 representing the cornea. Thus the eye is actually comprised of two somewhat modified spheres placed one in front of the other. The anterior of these two segments is the smaller more curved cornea.

The globe of the eye consists of three concentric coverings enclosing the various transparent media through which the light must pass before reaching the sensitive retina. The outermost covering is a fibrous protective portion, the posterior five-sixths of which is white and opaque and called the sclera 13, and sometimes referred to as the white of the eye where visible to the front. The anterior one-sixth of this outer layer is the transparent cornea 12.

A middle covering is mainly vascular and nutritive in function and is comprised of the choroid 14, ciliary body 15 and iris 17. The choroid generally functions to maintain the retina. The ciliary muscle is involved in suspending the lens and accommodation of the lens. The iris is the most anterior portion of the middle covering of the eye and is arranged in a frontal plane. It is a thin circular disc corresponding to the diaphragm of a camera, and is perforated near its center by a circular aperture called the pupil 19. The size of the pupil varies to regulate the amount of light which reaches the retina. It contracts also to accommodation, which serves to sharpen the focus by diminishing spherical aberration. The iris divides the space between the cornea 12 and the lens 21 into an anterior chamber 22 and posterior chamber 23. The innermost portion of covering is the retina 18, consisting of nerve elements which form the true receptive portion for visual impressions.

The retina is a part of the brain arising as an outgrowth from the fore-brain, with the optic nerve 24 serving as a fibre tract connecting the retina part of the brain with the fore-brain. A layer of rods and cones, lying just beneath a pigmented epithelium on the anterior wall of the retina, serve as visual cells or photoreceptors which transform physical energy (light) into nerve impulses.

The vitreous 26 is a transparent gelatinous mass which fills the posterior four-fifths of the globe. At its sides it supports the ciliary body 16 and the retina 18. A frontal saucer-shaped depression houses the lens 21.

The lens 21 of the eye is a transparent bi-convex body of crystalline appearance placed between the iris 17 and vitreous 26. Its axial diameter varies markedly with accommodation. A ciliary zonule 27, consisting of transparent fibers passing between the ciliary body 16 and lens 21 serves to hold the lens in position and enable the ciliary muscle to act on it.

Referring again to the cornea 12, this outermost fibrous transparent coating resembles a watch glass. Its curvature is somewhat greater than the rest of the globe and is ideally spherical in nature. However, often it is more curved in one meridian than another, giving rise to astigmatism. A central third of the cornea is called the optical zone with a slight flattening taking place outwardly thereof as the cornea thickens towards it periphery. Most of the refraction of the eye takes place on the surface of the cornea.

Referring next to Figure 2 of the drawings, the globe of an eye is shown having a cornea 12 with a normal curvature represented by the solid line 39. If parallel rays of light 41 pass through the corneal surface 39 of Figure 2, they are refracted by the corneal surfaces to converse eventually near the retina 18 of the eye. The diagram of Figure 2 discounts, for the purposes of this discussion, the refractive effect of the lens or other portions of the eye. The eye depicted in Figure 2 is hyperopic and thus the rays of light 41 are refracted to converge at point 42 behind the retina. If a peripheral band of pressure is applied inwardly at the chord 43 of the cornea, the walls of the cornea are caused to steepen. This is because the volume of fluids within the anterior chamber 22 remains constant, thus the anterior portion of the cornea, including the optical zone (inner third of the cornea) steepens in slope to form a curvature (shown in exaggeration) following the dotted line 44. The rays of light 41 are then refracted from the steeper surface 44 at a greater angle to direct the refracted rays into focus at shorter distance, such as directly on the retina 18.

Figure 3 shows a similar eye system to that of Figure 2 except that the so-called normal corneal curvature of Figure 3 causes the light rays 41 to refract into focus at a point 46 in the vitreous which is short of the retinal surface 18. This is typical of a myopic eye. If chord 43 of the cornea is expanded uniformly outwardly as shown by the arrows, the walls of the cornea are flattened. Light rays 41 refracted by the now-flattened corneal surface will be refracted at a smaller angle and thus converge at a more distant point such as directly on the retina 18.

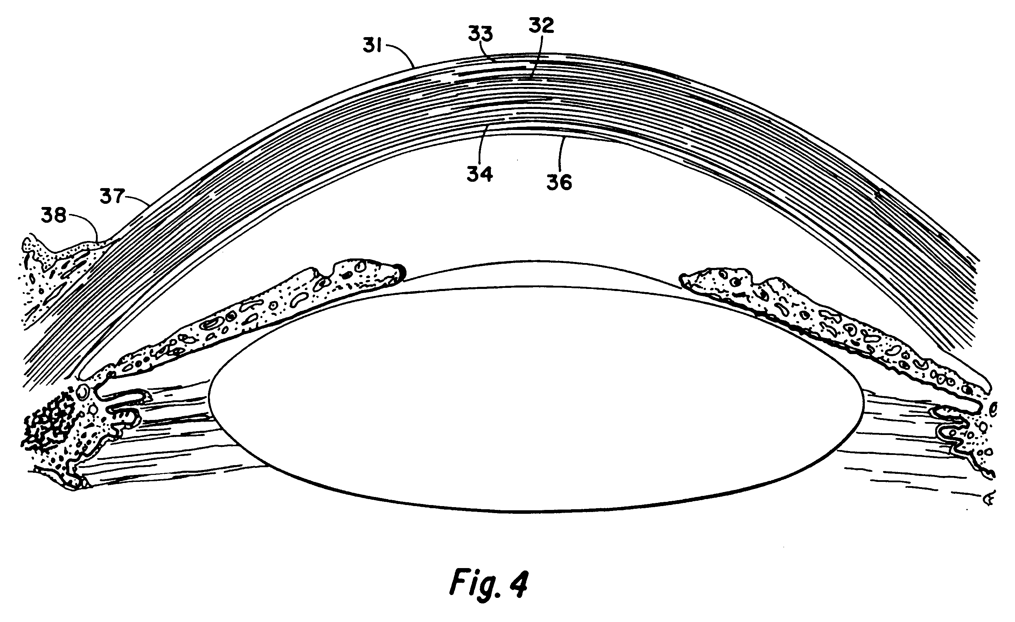

Referring now to Figure 4, a more detailed drawing of the anterior portion of the globe shows the various layers of the cornea comprising an epithelium 31. Epithelial cells on the surface thereof function to maintain transparency of the cornea. These epithelial cells are rich in glycogen, enzymes and acetylcholine and their activity regulates the corneal corpuscles and controls the transport of water and electrolytes through the lamellae of the stroma 32 of the cornea.

An anterior limiting lamina 33, referred to as Bowman's membrane, is positioned between the epithelium 31 and the substantia propria or stroma 32 of the cornea. The stroma is comprised of lamella having bands of fibrils parallel to each other and crossing the whole of the cornea. While most of the fibrous bands are parallel to the surface, some are oblique, especially anteriorly. The fibrous bands within alternate lamella are at a near right angle to bands in the adjacent lamella. A posterior limiting lamina 34 is referred to as Descemet's membrane. It is a strong membrane sharply defined from the stroma and resistant to pathological processes of the cornea.

The endothelium 36 is the most posterior layer of the cornea and consists of a single layer of cells. The limbus 37 is the transition zone between the conjunctiva 38 and sclera 13 on the one hand and the cornea 12 on the other.

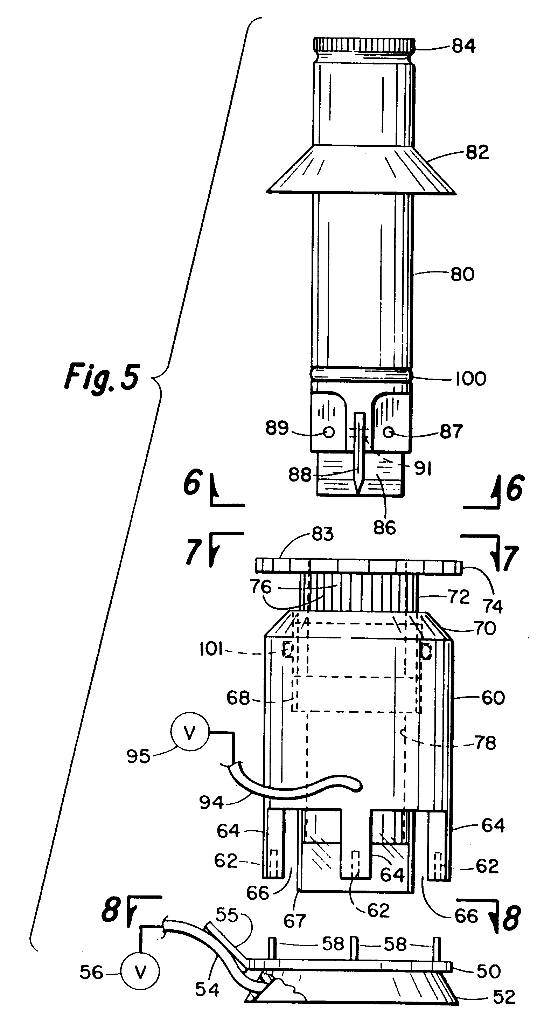

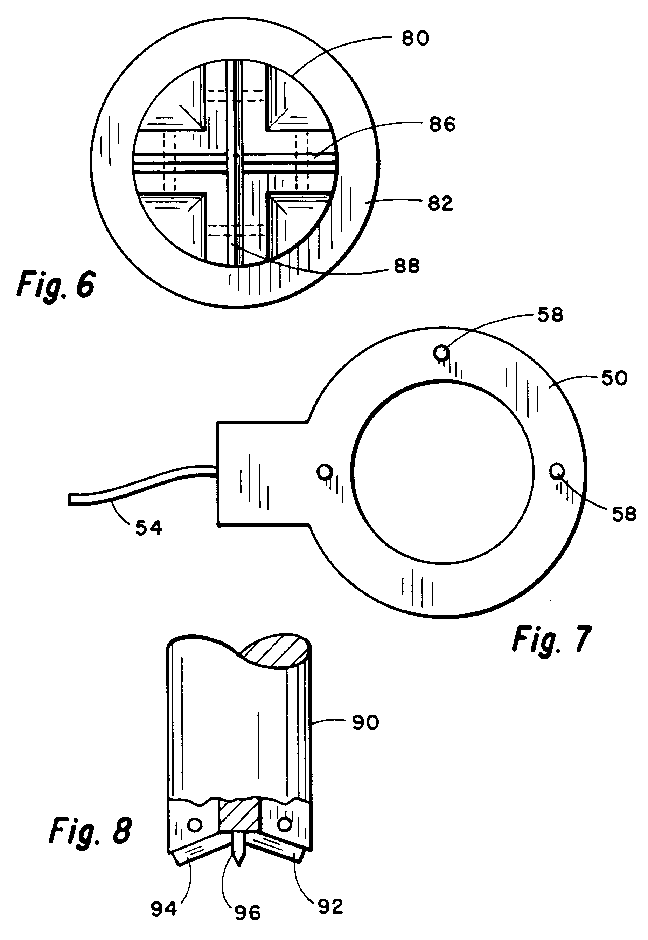

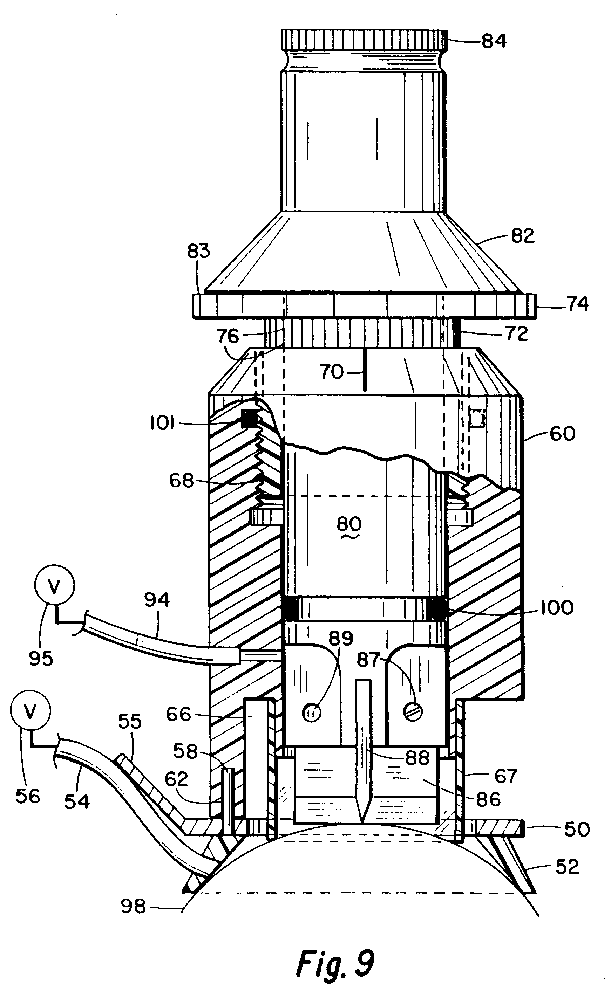

Referring now to Figures 5-9, the assembly of the basic parts of the apparatus are shown in exploded and assembled views. These parts comprises a cylindrical positioning ring 50 having a resilient vacuum ring 52 extending from the bottom side of the positioning ring for contact with the eye of the patient being treated. A vacuum hose 54 provides communication from the inside of the resilient ring 52 and a vacuum pump source means 56 as a means to retain the assembled parts upon the eye for surgical procedures herein described. A plurality of positioning pins 58 are provided on the top side of the positioning ring to receive the cylindrical holding sleeve 60, the pins being adapted to be received through openings 62 in legs 64 of holding sleeve 60. Visual inspection openings 66 are provided between the legs for use by the surgeon or technician performing the process. A translucent, flexible, e.g. vinyl, cylinder 67 is centrally positioned at the bottom of the holding sleeve, which, when in use, will contact the outer portion of the cornea. The interior of the cylindrical holding sleeve 60 includes a plurality of screw threads 68 along a portion of its length, the threads being a very fine thread, e.g. of a pitch equal to 40 threads per 2,5 cm (inch). An indicia or marker 70 is provided in the body of the cylindrical holding sleeve so as to provide a visual measuring point for the surgeon relative to the rotatable position of a micrometer-like guide sleeve 72 which includes exterior threads to match threads 68 of the cylindrical holding sleeve. The guide sleeve includes an outer knob portion 74 and indicia generally designated by the numeral 76, e.g. millimeter markings on the lower portion of the guide sleeve which function to provide the amount of axial movement of the guide sleeve. The interior surface 78 of the cylindrical holding sleeve is adapted to rotatably receive a scraping tool 80. A conduit 94 communicates the interior of the holding sleeve with a vacuum source 95. The scraping tool includes a collar 82 which is adapted to rest upon the top surface 83 of the guide sleeve 72 for movement upwardly or downwardly therewith. The top end of the scraping tool can include a knurled grip portion 84 for rotation and/or oscillation by the surgeon. Positioned along the body of the scraping tool is O-ring 100 to seal against interior surface 78. An upper O-ring 101 frictionally engages the threads of a guide sleeve 72 to prevent inadvertent turning. This, along with the cylinder 67, forms a vacuum chamber above the cornea 98. At the bottom of the scraper tool are a plurality of blades 86 and 88 which are retained within the body of the scraping tool 80 by pins 87, 89, and 91. The blades 86 and 88 are retained transverse to the longitudinal axis of the scraping tool 80. The blades 86 and 88 as used in the invention are of surgical steel. The scraping tool 80 of Figure 5 is adapted to provide a scraping operation upon the cornea over the top center thereof for myopia refractive error, i.e. near-sightedness, which will effectively lengthen the corneal radius of curvature as described relative to Figure 3. To correct for hyperopia (far-sightedness), the scraping tool blades as shown and described in Figure 8 of Serial No. 450,672 are utilized. The blades are adapted to contact the outer anterior portion of the cornea in order to shorten the effective radius thereof, that is, the blades will be adapted to contact the area A as shown in Figure 2 whereas the scraping tool 80 of Figure 5 will be adapted to sculpt the area B of Figure 3.

The operation of the apparatus is accomplished by first taking optical measurements of the eye as to the shape of the cornea and to determine the refractive error, for example, the shape the cornea should have in order for that eye to operate in an optically correct manner--i.e. correct refractive errors. Typically, a kerotograph photographic image using a placido-ring target such as described in U.S. Patent 3,797,921 is used. The photograph is of reflected light from the placido rings upon a standard spherical surface of the same size as the cornea in question, creating an image in the same manner as a topographic contour map. Subsequently, the topographic survey of the eye to be corrected is made for comparison purposes and to provide the surgeon with the necessary information for correcting the refractive errors. Once this occurs, the operation will proceed by placing the positioning ring 50 over the eye. The size of this ring may vary for different operations but is preferably of size wherein the resilient vacuum ring 52 will rest upon the sclera of the eye concentric about the cornea. Once the cylindrical positioning ring 50 is in place, the cylindrical holding sleeve 60 is then positioned thereupon by the engagement of openings 62 with positioning pins 58. Resilient vinyl seal tubing or cylinder 67 contacts the outer circumference of the cornea to seal therewith. Thereafter, the scraping tool 80 is inserted within the cylindrical holding sleeve 60 to a position where the bottom of the knife-edge blades 86 and 88 will initially contact the cornea. As shown in U.S. Application S.N. 450,672, and incorporated herein by reference, the determination of contact of the tool blades with the cornea can be achieved electrically. See Figure 11 of the aforesaid application. By rotating the guide sleeves 72 in incremental amounts as dictated by the caliper or measuring scales 70 and 76, the surgeon can continue to increase the depth of the scraping operation. Scraping of the cornea occurs by hand rotation of the scraping tool 80 although other mechanical or motor operated means are within the scope of this invention.

In myopic conditions, the scraping tool 80 of Figure 5 is utilized. During the operation, the knife-edge blades press upon the corneal surface which becomes depressed and thus gives a larger contact surface with the blades resulting in a larger diameter of sculptured surface. The scraping action is accentuated in proportion to the pressure between the cornea and the blade. With a partial vacuum formed in the chamber above the cornea, the cornea becomes less yieldable and semi-rigid. The extent of rigidity is a function of the amount of vacuum. Pressures between five-eighths (5/8) to three-fourths (3/4) bar (five-eighths (5/8) to three-fourths (3/4) atmosphere or six (6) to ten (10) inches of Mercury (Hg)) appear to be preferable, although not limiting to the purposes of this invention. It has been found that this allows the tool to have a greater positive 'feel' during the procedure with more predictable results in removal of corneal material to achieve the correct contour. The resulting effect is a lengthening of the refractive radius in that portion of the cornea under the blade. When the tool is removed, the cornea returns to its normal contour except that the radius over the top center thereof is now longer than it was initially. As a result, refractive light through the cornea now focuses upon the retina. The scraping action occurs by the surgeon in incremental movement by rotating the guide sleeve 72 relative to the cylindrical holding sleeve 60 utilizing the incremental measuring indicia 76 relative to pointer or other indicia 70. As one example, the guide sleeve is graduated into 25 micrometer divisions to provide .001" adjustments for each marked division of rotation. Through use, the surgeon or technician begins to decide the amount of downward movement needed to achieve the required changes in the cornea by the rotation and/or oscillation of the knives. The rotation for a period of a few seconds will result in removal of small amounts of corneal material from the cornea. The tool can be removed and/or kerotographic photographs taken to determine if the refractive error has been corrected. Since the apparatus deals with very small increments of movement in the corneal reprofiling process, it is essential that the first contact setting be precise and accurate. Many times this can be done by visual means by the surgeon and in other instances electrical detecting means as previously described can be provided between the cornea and the tool blade to provide an exact setting of the tool which permits repeatable amounts of corneal removal.

## Claims

1. Apparatus for correcting refractive errors in an eye, by surgically reprofiling the corneal portion of the eye to change its radius, said apparatus comprising a rotating profiling tool and means to rotate said tool relative to the eye cornea, characterized in that said profiling tool is a scraping tool having at least one sharpened knife edge blade, having a shape adapted to scrape said cornea to a desired corneal radius, and in that the apparatus further comprises means to create a vacuum chamber above said cornea and means to position said scraping tool within said vacuum chamber and to rotate or oscillate said scraping tool against said cornea so as to achieve said desired corneal radius.

2. Apparatus according to claim 1, characterized in that it includes indexing means to provide indication of the axial location of said scraping tool relative to said cornea.

3. Apparatus according to claim 1, characterized in that said means to create a vacuum chamber are adapted to create an amount of vacuum sufficient to make said cornea semi-rigid.

4. Apparatus according to claim 3, characterized in that said vacuum is within the range of 5/8 to 3/4 bar (6 to 10 inches of Hg).

5. Apparatus according to claim 1, characterized in that it comprises
a positioning ring (50) having means to be temporarily attached to and surround the eye relative to the cornea to be reprofiled;
a holding sleeve (60) having means (58) at its bottom to be retained by the position ring (50), a guide sleeve (72) rotatable to the holding sleeve (60),
means to create a vacuum chamber within said holding sleeve (60) above said cornea;
a rotatable scraping tool (80) adapted to be received within the holding sleeve (60) and supported by the guide sleeve (72), a bottom end of the scraping tool (80) being positionable within said vacuum chamber and comprised of sharpened knife edge blade means, at said bottom end, to scrape a portion of the cornea necessary to correct said refractive errors.

6. Apparatus according to claim 1, characterized in that said scraping tool is manually rotatable.

7. Apparatus according to claim 1, characterized in that said means to position the scraping tool are arranged to retain said knife edge blades relative to a top center visual axis of said cornea and to allow to rotate or oscillate said blades against said cornea coaxially with said top center visual axis.

8. Apparatus according to claim 1, characterized in that said means to position said knife edge blade are arranged to retain said edge tangentially to and against a top of said cornea at a visual axis and to allow said knife edge to rotate or oscillate about said visual axis so as to scrape said cornea.

9. Apparatus according to claim 5, characterized in that
said positioning ring (50) is a cylindrical positioning ring having resilient vacuum ring means (52) on its bottom side for temporary attachment to a sclera portion of said eye and surrounding said cornea (98) to be reprofiled, said positioning ring being provided with positioning pins (58) at a top side thereof, and with vacuum means (56) communicating with said vacuum ring means (52);
said holding sleeve (60) being cylindrical and being provided with means at a bottom of said holding sleeve to interconnect with said positioning pins (58), screw threads (68) of a given pitch formed on an interior portion of said holding sleeve (60), and further having a resilient cylindrical sleeve (67) at the bottom thereof in contact with said eye around said cornea;
said guide sleeve (72) having screw threads of said given pitch formed exteriorly thereof for axial movement by rotatable attachment with said screw threads (68) of said holding sleeve (60);
said scraping tool (80) being adapted to be rotatably and axially received within said holding sleeve (60) and said guide sleeve (72), a collar means (82) being provided on said scraping tool to rotatably support said scraping tool (80) upon said guide sleeve (72), at least one knife edge blade (86, 88) being provided at the bottom of said scraping tool (80);
said apparatus further comprising means, within said vacuum chamber, to position said knife edge blade (86, 88) tangentially to and against a top center visual axis of said cornea (98); and
means to rotate or oscillate said knife edge blade (86, 88) coaxially with said visual axis of said eye so as to scrape said cornea and correct said refractive errors.

10. Apparatus according to claim 9, characterized in that said knife edge blade is shaped so as to allow the corneal radius to be effectively decreased.

11. Apparatus according to claim 9, characterized in that said knife edge blade (86, 88) is shaped so as to allow the corneal radius to be effectively increased.

12. Apparatus according to claim 9, characterized in that said scraping tool (80) comprises a plurality of radial blades (86, 88) the bottom sharpended knife edges of which are transverse to the axis of said scraping tool (80).

13. Apparatus according to claim 9, characterized in that said holding sleeve (60) and said resilient cylindrical sleeve (67) are substantially transparent.

14. Apparatus according to claim 9, characterized in that said pitch of said screw threads (68) is between 35 to 50 threads per 2,5 cm (inch).

15. Apparatus according to claim 14, characterized in that said pitch of said screw threads (68) is 40 threads per 2,5 cm (inch).

16. Apparatus according to claim 9, wherein said holding sleeve (60) and said guide sleeve (72) include micrometer indexing means for incrementally measuring said axial movement of said scraping tool (80).

17. Apparatus according to claim 9 including means to axially advance said blade (86, 88) incrementally against said cornea (98) until said refractive error has been corrected.

18. Apparatus according to claim 1, characterized in that
said scraping tool (80) comprises a spindle having at one end thereof, constituting its inner end, knife-edge scraper blade means (86, 88) projecting endwise therefrom and extending outwardly from the spindle axis for scraping off portions of the anterior surface of the cornea by rotating or oscillation of the tool with the edge of the blade means in scraping engagement with said anterior surface;
means for holding the spindle for rotation on its axis and for axial movement with the spindle extending at its said inner end out of said holding means;
means for mounting said holding means concentrically with a visual axis of said cornea in fixed relation to the eye with the blade means at the inner end of the spindle in position for scraping engagement with the anterior surface of the cornea to scrape said surface in a circular pattern about said visual axis;
means to create a vacuum in a space above said cornea; and
means for gauging the extension of the inner end of the spindle from the holding means to gauge the penetration of the edge of the blade means into the cornea for said scraping, said gauging means being adjustable to vary the extension of the inner end of the spindle from the holding means.

## Patentansprüche

1. Vorrichtung zur Korrektur von Brechungsfehlern in einem Auge durch chirugisches Reprofilieren des Hornhautbereichs des Auges zur Änderung dessen Radius, wobei die Vorrichtung ein rotierendes Profilierungswerkzeug und Mittel aufweist, um das Werkzeug relativ zu der Hornhaut des Auges zu drehen**, dadurch gekennzeichnet**, daß das Profilierungswerkzeug als Schabwerkzeug ausgeführt ist, das mindestens eine geschärfte Schneide einer Messerklinge mit einer zum Schaben der Hornhaut auf einen gewünschten Hornhautradius geeigneten Form aufweist und daß die Vorrichtung des weiteren Mitte] zur Erzeugung einer Vakuumkammer oberhalb der Hornhaut und Mittel zur Positionierung des Schabwerkzeuges innerhalb der Vakuumkammer und zur Drehung oder Oszillierung des Schabwerkzeuges gegen die Hornhaut aufweist, um den gewünschten Hornhautradius zu erhalten.

2. Vorrichtung nach Anspruch 1, **dadurch gekennzeichnet**, daß es Mittel zur Einteilung beinhaltet, um eine Anzeige der axialen Stellung des Schabwerkzeuges relativ zu der Hornhaut zu ermöglichen.

3. Vorrichtung nach Anspruch 1, **dadurch gekennzeichnet**, daß die Mittel zur Erzeugung der Vakuumkammer geeignet sind, ein Vakuum in ausreichender Höhe zu erzeugen, um eine halbstarre Hornhaut herzustellen.

4. Vorrichtung nach Anspruch 3, **dadurch gekennzeichnet**, daß der Unterdruck innerhalb des Bereiches von 5/8 bis 3/4 bar (6 bis 10 inches Hg) liegt.

5. Vorrichtung nach Anspruch 1, **dadurch gekennzeichnet**, daß vorgesehen sind:
- ein Positionierungsring (50) mit Mitteln zur zeitweiligen Anbringung an dem Auge und das Auge bezüglich der zu reprofilierenden Hornhaut umgebend,
- eine Halterungshülse (60) mit Mitteln (58) an ihrer Basis, die durch den Positionierungsring (50) zurückhaltbar sind,
- eine bezüglich der Halterungshülse (60) drehbare Führungshülse (72),
- Mittel zur Erzeugung einer Vakuumkammer innerhalb der Halterungshülse (60) oberhalb der Hornhaut,
- ein drehbares Schabwerkzeug (80), das innhalb der Halterungshülse (60) aufnehmbar ist und durch die Führungshülse (72) unterstützt ist, wobei der Basisgrund des Schabwerkzeugs (80) innerhalb der Vakuumkammer positionierbar ist und klingenartige Mittel mit geschärften Schneiden am Basisgrund aufweist, um einen zur Korrektur des Brechungsfehlers notwendigen Bereich der Hornhaut abzuschaben.

6. Vorrichtung nach Anspruch 1, **dadurch gekennzeichnet**, daß das Schabwerkzeug manuell drehbar ist.

7. Vorrichtung nach Anspruch 1, **dadurch gekennzeichnet**, daß die Mittel zur Positionierung des Schabwerkzeugs so angeordnet sind, daß die Schneiden der Messerklingen relativ zu der Gesichtsachse des oberen Zentrums der Hornhaut zurückhaltbar sind und eine Drehung oder Oszillierung der Klingen gegen die Hornhaut koaxial mit der Gesichtsachse des oberen Zentrums ermöglicht ist.

8. Vorrichtung nach Anspruch 1, **dadurch gekennzeichnet**, daß die Mittel zur Positionierung der Schneide der Messerklinge so angeordnet sind, daß die Schneide tangential zu und gegen einen Scheitel der Hornhaut auf einer Gesichtsachse haltbar ist und eine Drehung oder Oszillierung der Messerschneide um die Gesichtsachse ermöglicht ist, um so die Hornhaut abzuschaben.

9. Vorrichtung nach Anspruch 5, **dadurch gekennzeichnet**,
- daß der Positionierungsring (50) als zylindrischer Positionierungsring ausgeführt ist, der einen elastischen Vakuumring (52) auf seiner Basisseite zur zeitweiligen Anbringung an einem Bereich der Lederhaut des Auges aufweist und die zu reprofilierende Hornhaut (98) umgibt, wobei der Positionierungsring auf seiner Oberseite mit Positionierungszapfen (58) und mit Mitteln zur Erzeugung eines Vakuums (56) versehen ist, die mit dem Vakuumring (52) in Verbindung stehen,
- daß die Halterungshülse (60) zylindrisch ausgeführt ist und an ihrer Basis mit Mitteln zur Verbindung mit den Positionierungszapfen (58) versehen ist, wobei ein Schraubgewinde (68) einer vorgegebenen Ganghöhe auf einem inneren Teilbereich der Halterungshülse (60) ausgebildet ist, und die des weiteren eine elastische zylindrische Hülse (67) an der Basis derselben in Kontakt mit dem Auge um die Hornhaut aufweist,
- daß die Führungshülse (72) außenseitig ein angeordnetes Schraubgewinde der gegebenen Ganghöhe zur axialen Bewegung durch drehbare Anbringung mit dem Schraubgewinde (68) der Halterungshülse (60) aufweist,
- daß das Schabwerkzeug (80) drehbar und axial innerhalb der Halterungshülse (60) und der Führungshülse (72) aufnehmbar ist, daß Anschlußmittel (82) an dem Schabwerkzeug vorgesehen sind, um das Schabwerkzeug (80) drehbar auf der Führungshülse (72) zu unterstützen und daß zumindest eine Schneide einer Messerklinge (86, 88) an der Basis des Schneidwerkzeuges (80) vorgesehen ist,
- daß die Vorrichtung des weiteren innerhalb der Vakuumkammer Mittel zur Positionierung der Schneide der Messerklingen (86, 88) tangential zu und gegen die Gesichtsachse des oberen Zentrums der Hornhaut (98) aufweist und
- daß Mittel zur Drehung oder Oszillierung der Schneiden der Messerklingen (86, 88) koaxial mit der Gesichtsachse des Auges vorgesehen sind, um so die Hornhaut abzuschaben und den Brechungsfehler zu korrigieren.

10. Vorrichtung nach Anspruch 9, **dadurch gekennzeichnet**, daß die Schneide der Messerklingen (86, 88) so geformt ist, daß eine wirksame Verringerung des Radius der Hornhaut ermöglicht ist.

11. Vorrichtung nach Anspruch 9, **dadurch gekennzeichnet**, daß die Schneide der Messerklingen (86, 88) so geformt ist, daß eine wirksame Erhöhung des Radius der Hornhaut ermöglicht ist.

12. Vorrichtung nach Anspruch 9, **dadurch gekennzeichnet**, daß das Schabwerkzeug (80) eine Vielzahl von radialen Klingen (86, 88) aufweist, wobei die an ihrer Basis geschärften Schneiden derselben quer zu der Achse des Schabwerkzeuges (80) verlaufen.

13. Vorrichtung nach Anspruch 9, **dadurch gekennzeichnet**, daß die Halterungshülse (60) und die elastische, zylindrische Hülse (67) im wesentlichen transparent ausgeführt sind.

14. Vorrichtung nach Anspruch 9, **dadurch gekennzeichnet**, daß die Ganghöhe des Schraubgewindes (68) zwischen 35 und 50 Windungen je 2,5 cm (inch) beträgt.

15. Vorrichtung nach Anspruch 14, **dadurch gekennzeichnet**, daß die Ganghöhe des Schraubgewindes (68) 40 Windungen je 2,5 cm (inch) beträgt.

16. Vorrichtung nach Anspruch 9, **dadurch gekennzeichnet**, daß die Halterungshülse (60) und die Führungshülse (72) Mittel zur Mikrometereinteilung zur inkrementweisen Messung der axialen Bewegung des Schabwerkzeuges (80) beinhalten.

17. Vorrichtung nach Anspruch 9, die Mittel zum axialen, inkrementweisen Vorrücken der Messerklinge (86, 88) gegen die Hornhaut (98) beinhaltet, bis daß der Brechungsfehler korrigiert ist.

18. Vorrichtung nach Anspruch 1, **dadurch gekennzeichnet**,
- daß das Schabwerkzeug (80) eine Welle aufweist, die an einem Ende derselben, welches das innere Ende darstellt, klingenartige Mittel zum Schaben (86, 88) aufweist, die aufrecht von dieser hervorstehen und sich auswärtsgewandt von der Wellenachse erstrecken, um Bereiche der vorderen Oberfläche der Hornhaut durch Drehung oder Oszillierung des Werkzeuges unter schabendem Angriff der Klinge des Schneidmittels mit der vorderen Oberfläche abzuschaben,
- daß Mittel zur Halterung der Welle bei der Drehung um ihre Achse und zur axialen Bewegung der mit an ihrem inneren Ende aus dem Halterungsmittel hervorstehender Welle vorgesehen sind,
- daß Mittel zur Befestigung der Halterungsmittel konzentrisch zur Gesichtsachse der Hornhaut in fester Beziehung zu dem Auge vorgesehen sind, wobei die klingenartigen Mittel an dem inneren Ende der Welle in einer Stellung zum schabenden Angriff mit der vorderen Oberfläche der Hornhaut angeordnet sind, um die Oberfläche in einem ringförmigen Muster um die Gesichtsachse zu schaben,
- daß Mittel zur Erzeugung eines Vakuums in einem Raum oberhalb der Hornhaut vorgesehen sind, und
- daß Mittel zur Vermessung der Ausdehnung des inneren Endes der Welle von dem Halterungsmittel vorgesehen sind, um die Eindringung der Schneide der klingenartigen Mittel in die Hornhaut zum Abschaben derselben zu vermessen, wobei die Vermessungsmittel einstellbar sind, um die Ausdehnung des inneren Endes der Welle von den Halterungsmitteln zu variieren.

## Revendications

1. Appareil pour corriger les erreurs de réfraction dans un oeil, par rectification chirurgicale du profil de la partie cornéale de l'oeil, de façon à en modifier le rayon de courbure, ledit appareil comportant un outil de profilage rotatif et des moyens pour faire tourner cet outil par rapport à la cornée de l'oeil, caractérisé en ce que ledit outil de profilage est un outil racleur ayant au moins une lame de couteau à arête aiguisée, ayant une forme adaptée pour racler ladite cornée jusqu'à un rayon de courbure cornéen désiré, et en ce que l'appareil comprend, en outre, des moyens pour créer une chambre à vide au-dessus de ladite cornée et des moyens pour positionner ledit outil racleur à l'intérieur de ladite chambre à vide et faire tourner ou osciller cet outil racleur contre ladite cornée de façon à obtenir ledit rayon de courbure désiré.

2. Appareil selon la revendication 1, caractérisé en ce qu'il comprend des moyens de repérage pour fournir des indications concernant la position axiale dudit outil racleur par rapport à ladite cornée.

3. Appareil selon la revendication 1, caractérisé en ce que lesdits moyens pour créer une chambre à vide sont appropriés à la production d'un vide suffisant pour rendre ladite cornée semi-rigide.

4. Appareil selon la revendication 3, caractérisé en ce que ledit vide est compris dans le domaine de 5/8 à 3/4 bar (6 à 10 pouces de mercure).

5. Appareil selon la revendication 1, caractérisé en ce qu'il comprend
un anneau de positionnement (50) ayant des moyens pour le fixer, de manière temporaire, sur l'oeil en entourant celui-ci par rapport à la cornée dont le profil doit être rectifié;
un manchon support (60) muni, à sa partie inférieure, de moyens de rétention par l'anneau de positionnement (50), un manchon de guidage (72) apte à tourner par rapport au manchon support (60);
des moyens pour créer une chambre à vide à l'intérieur dudit manchon support (60) au dessus de ladite cornée;
un outil racleur rotatif (80) agencé de manière à pouvoir être logé dans le manchon support (60) et être supporté par le manchon de guidage (72), une extrémité inférieure de l'outil racleur (80), étant agencée de manière à pouvoir être positionnée à l'intérieur de ladite chambre à vide et comprenant des moyens formant lame de couteau à arête aiguisée, à ladite extrémité inférieure, pour racler une partie de la cornée nécessaire pour corriger lesdites erreurs de réfraction.

6. Appareil selon la revendication 1, caractérisé en ce que ledit outil racleur peut être mis en rotation manuellement.

7. Appareil selon la revendication 1, caractérisé en ce que lesdits moyens pour positionner l'outil racleur sont agencés de manière à maintenir lesdites lames de couteau par rapport à un axe optique central supérieur de ladite cornée et à permettre de faire tourner ou osciller ces lames contre ladite cornée coaxiallement audit axe optique central supérieur.

8. Appareil selon la revendication 1, caractérisé en ce que lesdits moyens de positionnement desdites lames de couteau sont agencés de manière à maintenir ladite arête tangentiellement par rapport à la surface de ladite cornée et contre cette surface, sur un axe optique, et à permettre à ladite lame de couteau de tourner ou osciller autour dudit axe optique, de façon à racler ladite cornée.

9. Appareil selon la revendication 5, caractérisé en ce que
ledit anneau de positionnement (50) est un anneau de positionnement cylindrique ayant des moyens de vide annulaires, élastiques (52), sur son côté inférieur, pour fixation temporaire sur une partie de la sclérotique dudit oeil et entourant ladite cornée (98) dont le profil doit être corrigé, ledit anneau de positionnement (58), sur un côté supérieur de celui-ci, et de moyens de vide (56) communiquant avec lesdits moyens de vide annulaires (52);
ledit manchon support (60) étant cylindrique et étant muni de moyens d'interconnexion avec lesdites chevilles de positionnement (58), placés au bas dudit manchon support, d'un filetage d'un pas déterminé, formé sur une partie intérieure dudit manchon support (60) et ayant, en outre, à sa partie inférieure, un manchon cylindrique élastique (67) en contact avec ledit oeil autour de ladite cornée;
ledit manchon de guidage (72) ayant un filetage dudit pas déterminé formé sur une partie extérieure de ce manchon pour permettre un déplacement axial par mise en prise rotative avec ledit filetage (68) dudit manchon support (60);
ledit outil racleur (80) étant agencé de façon à pouvoir être logé dans ledit manchon support (60) et ledit manchon de guidage (72), des moyens en forme de collerette (82) étant agencés sur ledit outil racleur pour supporter rotativement cet outil racleur (80) sur ledit manchon de guidage (72) au moins un lame à arête de couteau (86, 88) étant disposée à la partie inférieure dudit outil racleur (80);
ledit appareil comprenant en outre, à l'intérieur de ladite chambre à vide, des moyens pour positionner ladite lame à arête de couteau (86, 88) tangentiellement à, et contre, un axe optique central supérieur de ladite cornée (98); et des moyens pour faire tourner ou osciller ladite lame de couteau (86, 88) coaxiallement audit axe optique dudit oeil, de façon à racler ladite cornée et corriger lesdites erreurs de réfraction.

10. Appareil selon la revendication 9, caractérisé en ce que ladite lame de couteau est conformée de manière à permettre de diminuer efficacement le rayon de courbure de la cornée.

11. Appareil selon la revendication 9, caractérisé en ce que ladite lame de couteau (86, 88) est conformée de manière à permettre d'augmenter efficacement le rayon de courbure de la cornée.

12. Appareil selon la revendication 9, caractérisée en ce que ledit outil racleur (80) comprend une pluralité de lames radiales (86, 88) dont les parties inférieures aiguisées en arêtes de couteau sont transversales par rapport à l'axe dudit outil racleur (80).

13. Appareil selon la revendication 9, caractérisé en ce que ledit manchon support (60) et ledit manchon cylindrique élastique (67) sont essentiellement transparents.

14. Appareil selon la revendication 9, caractérisé en ce que ledit pas dudit filetage (68) est compris entre 35 et 50 filets par 2,5 cm (pouce).

15. Appareil selon la revendication 14, caractérisé en ce que ledit pas dudit filetage (68) est de 40 filets par 2,5 cm (pouce).

16. Appareil selon la revendication 9, caractérisé en ce que ledit manchon support (60) et ledit manchon de guidage (72) comprennent des moyens de repérage micrométriques pour mesurer, par accroissements, ledit mouvement axial dudit outil racleur (80).

17. Appareil selon la revendication 9, comprenant des moyens pour faire avancer ladite lame (86, 88) par accroissements contre ladite cornée (98) jusqu'à ce que ladite erreur de réfraction ait été corrigée.

18. Appareil selon la revendication 1, caractérisé en ce que ledit outil racleur (80) comprend une broche ayant, à l'une des ses extrémités, constituant son extrémité intérieure, des moyens de raclage (86, 88) à lame de couteau en saillie à son extrémité et s'étendant vers l'extérieur, à partir de l'axe de la broche, pour enlever par raclage des parties de la surface antérieure de la cornée par rotation ou oscillation de l'outil, avec l'arête des moyens en forme de lame en prise de raclage avec ladite surface antérieure;
des moyens pour supporter la broche en permettant sa rotation sur son axe et son déplacement axial avec la broche s'étendant à ladite extrémité intérieure à l'extérieur desdits moyens de support;
des moyens pour monter lesdits moyens de support concentriquement par rapport à un axe optique de ladite cornée, en position fixe par rapport à l'oeil avec les moyens formant lame, à l'extrémité intérieure de la broche, en position pour la mise en prise de raclage avec la surface antérieure de la cornée, de façon à racler cette surface selon une configuration circulaire autour dudit axe optique;
des moyens pour produire un vide dans un espace situé au dessus de ladite cornée; et
des moyens pour jauger le degré d'extension de l'extrémité intérieure de la broche, à partir des moyens de support, pour la détermination de la pénétration de l'arête des moyens formant lame dans la cornée, pour ledit raclage, lesdits moyens de jauge étant réglables pour faire varier le degré d'extension de l'extrémité intérieure de la broche à partir des moyens de support.
